Europäisches Patentamt

European Patent Office

Office européen des brevets

(19)

(11) Numéro de publication : **0 003 205**
**B1**

# (12) FASCICULE DE BREVET EUROPÉEN

(45) Date de publication du fascicule du brevet :
25.03.81

(21) Numéro de dépôt : **79420001.4**

(22) Date de dépôt : **10.01.79**

(51) Int. Cl.³ : **C 07 C 69/00,** C 07 C 67/04,
C 07 C 69/14

(54) **Procédé pour la préparation d'esters d'éthyle.**

(30) Priorité : **16.01.78 FR 7801570**
**16.01.78 FR 7801570**

(43) Date de publication de la demande :
**25.07.79 (Bulletin 79/15)**

(45) Mention de la délivrance du brevet :
**25.03.81 Bulletin 81/12**

(84) Etats contractants désignés :
**BE CH DE GB IT LU NL SE**

(56) Documents cités :
**US - A - 3 474 131**

(73) Titulaire : **RHONE-POULENC INDUSTRIES**
**22, avenue Montaigne**
**F-75008 Paris (FR)**

(72) Inventeur : **Gruffaz, Max**
**6, Avenue du Général de Gaulle**
**F-69350 La Mulatiere (FR)**
Inventeur : **Micaelli, Odile**
**11, rue Montvert**
**F-69008 Lyon (FR)**

(74) Mandataire : **Rioufrays, Roger et ai**
**RHONE-POULENC INDUSTRIES Centre de Recherches des Carrières Service Brevets**
**F-69190 Saint-Fons (FR)**

EP 0 003 205 B1

Imprimerie Jouve, 17, rue du Louvre, 75001 Paris, France

## 0 003 205

### Procédé pour la préparation d'esters d'éthyle

La présente invention a trait à un procédé pour la préparation d'esters d'éthyle. Elle a plus spécialement pour objet un procédé de préparation d'esters d'éthyle, par réaction d'un acide carboxylique sur l'éthylène.

Depuis de nombreuses années, il est connu que les acides carboxyliques s'additionnent sur les oléfines, en présence de catalyseurs acides. Si les avantages de cette méthode de synthèse d'esters, par rapport aux procédés conventionnels faisant intervenir en phase liquide des alcools et des catalyseurs acides minéraux, ont été reconnus rapidement, cette méthode, notamment dans le cas où l'on cherche à obtenir des esters d'éthyle, est restée une curiosité de laboratoire, en raison des difficultés rencontrées.

En effet, comme le montrent des travaux antérieurs (Ind. & Eng. Chem. 1951, 43, p 1596-1600 — et J. appl. Chem. 1963, p 544-547) l'éthylène ne peut être estérifié par un acide carboxylique, en présence d'un catalyseur acide, que dans des conditions extrêmement sévères. Ces conditions extrêmement dures favorisent les réactions secondaires, en particulier les réactions de polymérisation, qui se produisent au détriment de l'ester recherché.

Un but de la présente invention est de fournir un procédé perfectionné pour la préparation d'esters d'éthyle, par réaction de l'éthylène sur des acides carboxyliques, avec un rendement accru. Un autre but de l'invention, est de réaliser l'estérification de l'éthylène connue pour être difficile, dans des conditions beaucoup moins sévères, et de rendre ainsi le procédé plus économique, sur le plan industriel.

Les recherches, qui ont abouti à la présente invention, ont montré d'une manière en soi remarquable, que l'on peut faire réagir de l'éthylène et au moins un acide carboxylique, en phase liquide, en présence d'au moins un catalyseur acide, pour obtenir des esters d'éthyle avec un bon rendement, en opérant dans un certain type de solvants, défini ci-après, dans des conditions relativement douces, de telle sorte que le processus puisse être appliqué aisément à l'échelle industrielle.

Des recherches ont montré que c'est la combinaison remarquable de l'utilisation d'un catalyseur acide et de la présence de ce type particulier de solvants, qui permet de réaliser ce progrès, et, par opposition à l'art antérieur, avec succès pour divers acides carboxyliques.

Il a donc maintenant été trouvé que l'on peut préparer des esters d'éthyle, à partir d'éthylène, en faisant réagir, en phase liquide de l'éthylène sur un mélange comprenant essentiellement un acide carboxylique, un catalyseur acide et au moins un solvant dissociant et capable d'augmenter l'acidité du milieu. En outre, le solvant doit être stable dans les conditions de la réaction, notamment en milieu acide et à haute température. D'autre part, le solvant doit être inerte dans les conditions de la réaction : il ne doit réagir lui-même, ni sur les réactifs, ni sur les produits. On a constaté que des composés organiques non basiques dont la constante diélectrique est au moins de l'ordre de 25 conviennent particulièrement bien à la mise en œuvre du procédé selon l'invention. A titre d'exemples de solvants utilisables, dans le cadre de la présente invention, on peut citer le nitrobenzène, la diméthylsulfone, la tétraméthylène sulfone ainsi que ses dérivés tels la 3-méthyl tétraméthylène sulfone, la 2,4-diméthyl tétraméthylène sulfone, et la dichloro tétraméthylène sulfone. La tétraméthylène sulfone et ses dérivés conviennent particulièrement bien à la mise en œuvre du procédé selon l'invention.

La présente invention envisage également les mélanges de tels solvants, dans la mesure où ces derniers sont compatibles. On peut utiliser notamment les mélanges de la tétraméthylène sulfone et de ses dérivés. On opère dans un milieu sensiblement anhydre.

Le choix de l'acide carboxylique à mettre en œuvre, selon la présente invention, dépendra bien évidemment de la nature de l'ester d'éthyle recherché. A titre d'exemples d'acides carboxyliques utilisables dans le cadre de la présente invention, on peut citer : les monoacides aliphatiques ayant jusqu'à 20 atomes de carbone dans la molécule, saturés ou insaturés, pouvant porter des substituants, notamment un ou plusieurs atomes d'halogènes, en particulier les acides acétique, propionique, butyrique, isobutyrique, hexanoïque, monochloroacétique, dichloroacétique, bromacétique, chloropropionique, acrylique et méthacrylique. L'invention envisage également les monoacides aromatiques, notamment les acides benzoïque et toluiques, les acides alicycliques tels les acides naphténiques, les diacides aliphatiques ayant de 3 à 6 atomes de carbone dans la molécule, notamment les acides succinique et adipique, et les diacides aromatiques, notamment les acides phtaliques. De préférence on opère avec l'acide acétique.

Les catalyseurs à mettre en œuvre, dans le cadre du procédé selon l'invention, sont des acides minéraux ou des acides organiques forts. A titre d'exemples de catalyseurs utilisables dans le cadre de la présente invention, on peut citer : l'acide sulfurique, les acides alcane-sulfoniques, notamment l'acide méthanesulfonique et ses homologues supérieurs jusqu'en C 6 ; les acides arylsulfoniques, en particulier l'acide benzènesulfonique, l'acide p-toluène-sulfonique, les acides benzènedisulfoniques, les acides naphtalènedisulfoniques ; l'acide fluorosulfonique ; les acides alcoylhalogènosulfoniques, en particulier les acides polyfluoroalcanesulfoniques et les acides perfluoroalcanesulfoniques ayant au plus 8 atomes de carbone dans la molécule.

L'invention envisage également l'utilisation comme catalyseurs de ces acides sous la forme de leurs esters d'alcoyles inférieurs ayant notamment de 1 à 4 atomes de carbone dans le radical alcoyle, et plus particulièrement sous la forme de leurs esters d'éthyle.

L'invention envisage également les mélanges de tels catalyseurs.

2

Selon une variante préférée de l'invention on utilise comme catalyseur acide un acide ou un mélange de plusieurs acides perfluoroalcanesulfoniques ayant au plus 8 atomes de carbone dans la molécule, le cas échéant, partiellement ou totalement sous la forme de leurs esters d'alcoyles inférieurs ayant, notamment de 1 à 4 atomes de carbone dans le radical alcoyle, les esters d'éthyle étant plus particulièrement envisagés.

L'acide trifluorométhanesulfonique et/ou ses esters d'alcoyles inférieurs ayant notamment de 1 à 4 atomes de carbone dans le radical alcoyle convient particulièrement bien à la mise en œuvre du procédé selon l'invention. L'acide trifluorométhanesulfonique et/ou son ester d'éthyle est plus spécialement envisagé.

Le procédé selon l'invention est particulièrement avantageux pour la préparation de l'acétate d'éthyle à partir d'éthylène et d'acide acétique, notamment lorsque l'on utilise la tétraméthylènesulfone et/ou ses dérivés, comme solvant. Outre son efficacité, le présent procédé présente l'avantage selon lequel tout ou partie du milieu réactionnel peut être recyclé.

En effet, en fin de réaction, on peut séparer l'acétate d'éthyle par tout moyen approprié et recycler tout ou partie du catalyseur, du solvant et le cas échéant de l'acide acétique qui n'a pas réagi. Les recherches qui ont abouti à la présente invention ont montré que l'efficacité du procédé en cause n'est sensiblement pas affectée par le fait que tout ou partie du catalyseur et du solvant provient d'un recyclage. En fin d'une première opération on peut séparer l'acétate d'éthyle formé par tout moyen approprié, en vue de sa récupération et recycler, dans une opération subséquente, le mélange restant sans qu'il soit nécessaire de séparer les constituants du mélange et/ou de les purifier au préalable. Le mélange, ainsi obtenu, renferme essentiellement le catalyseur, le solvant et éventuellement, l'acide acétique qui n'a pas réagi.

Lorsque dans une opération subséquente, l'éthylène est mis à réagir sur un tel mélange, en présence d'acide acétique une nouvelle quantité d'acétate d'éthyle est obtenue avec un rendement sensiblement égal à celui observé dans la première opération, et cela même lorsque plusieurs recyclages successifs sont réalisés.

Selon une variante préférée de l'invention, on ajoute lors du recyclage, une quantité d'acide acétique frais correspondant sensiblement à la quantité transformée dans l'opération antérieure.

On peut introduire l'acide acétique frais dans le courant de recyclage, ou directement dans le réacteur, par exemple.

Selon le procédé de l'invention, on obtient des esters d'éthyle, par réaction en phase liquide, de l'éthylène, sur un mélange comprenant essentiellement au moins un acide carboxylique, au moins un catalyseur acide, et au moins un solvant dont les caractéristiques ont été définies précédemment.

Selon une variante préférée de l'invention, la quantité relative de solvant, définie par la fraction molaire f de solvant dans le mélange de solvant et d'acide carboxylique-acide, constituant la fraction acide de l'ester d'éthyle recherché est supérieure ou égale à 0,4.

La quantité de catalyseur à introduire dans le milieu réactionnel n'est pas critique. En général, une quantité comprise entre 0,05 et 2 moles par litre de mélange réactionnel est satisfaisante. De préférence, cette quantité est comprise entre : 0,1 et 1 mole/litre.

La pression de l'éthylène doit être supérieure à la pression atmosphérique. Une pression comprise entre 20 et 60 bars convient particulièrement bien à la mise en œuvre de l'invention.

La température de réaction n'est pas critique. Toutefois, en dessous de 100° C, la vitesse de réaction est faible, alors qu'au-dessus de 200° C on constate l'apparition de produits secondaires par décomposition. De préférence, la température sera comprise entre 130 et 160° C.

Les exemples ci-après illustrent la mise en œuvre du procédé selon l'invention.

Exemples 1 à 7

Dans une bombe de 250 cm$^3$, en Hastelloy C, on charge de l'acide acétique, de la tétraméthylènesulfone (TMS) et un catalyseur. On porte à la température choisie dans un four à agitation longitudinale. Puis on envoie de l'éthylène. La pression de l'éthylène est de 40 bars sauf indications contraires. Au bout de 4 heures sauf indications contraires, on arrête la réaction. Les résultats obtenus, exprimés par le taux de transformation de l'acide en acétate d'éthyle (TT), ainsi que les conditions particulières sont donnés dans le tableau I, ci-après ; dans la dernière colonne, on a indiqué le taux de transformation de l'acide en acétate d'éthyle (TT$_0$) obtenu toutes conditions égales par ailleurs, en l'absence de tétraméthylènesulfone.

N.B. Dans ces exemples, ainsi que dans tous les exemples ci-après, la sélectivité en ester recherché est de l'ordre de 100 %.

TABLEAU I

| n° Exemple | Catalyseur Nature | Mole/litre | TMS en Mole | Acide acétique Mole | Température °C | TT % | $TT_o$ % |
|---|---|---|---|---|---|---|---|
| 1 | $H_2SO_4$ | 0,925 | 0,821 | 0,353 | 150 | 33,4 | 3,5 |
| 2 | $CH_3SO_3H$ | 1,00 | 0,822 | 0,345 | 160 | 16,5 | $\varepsilon$ |
| 3 | ⬡ avec —$SO_3H$ et $SO_3H$ | 0,50 | 0,793 | 0,342 | 160 | 50,7 | 4,9 |
| 4 | $CF_3SO_3H$ | 0,326 | 0,6 | 0,6 | 150 | 75,0 | 9,3 |
| 5* | $C_6F_{13}SO_3H$ | 0,158 | 0,604 | 0,601 | 160 | 54,2 | — |
| 6** | Résine | 10 g | 0,60 | 0,608 | 160 | 25,0 | — |
| 7 | $CF_3SO_3C_2H_5$ | 0,322 | 0,60 | 0,60 | 150 | 80,0 | — |

\* Cet essai a été réalisé avec une pression d'éthylène de 30 bars pendant 2 h 30 mn.

\*\* Cet essai a été réalisé avec 10 g d'une résine perfluorosulfonique commercialisée sous la marque NAFION - (Durée de l'essai : 3 heures). Cette résine renferme des copolymères du perfluoroéthylène et d'un éther perfluorovinylique - ledit éther portant des groupements acides sulfoniques.

Dans la dernière colonne de ce tableau ($TT_o$ %) :
$\varepsilon$ signifie : négligeable
— signifie : non déterminé

## Exemples 8 à 14

Selon le processus général décrit précédemment, on charge : de l'acide acétique, de l'acide trifluorométhanesulfonique, et un solvant.

Les conditions particulières ainsi que les résultats obtenus figurent dans le tableau II ci-après, dans lequel :

MTMS désigne la 3-méthyltétraméthylène sulfone
DMTMS désigne la 2,4-diméthyltétraméthylène sulfone
DMS désigne la diméthylsulfone
NB désigne le nitrobenzène
TMS désigne la tétraméthylènesulfone

TABLEAU II

| N° Ex. | $CF_3SO_3H$ (Mole/litre) | Solvant Nature | Mole | Acide acétique (Mole) | Pression (bars) | Température (°C) | Durée (h) | TT (%) |
|---|---|---|---|---|---|---|---|---|
| 8 | 0,313 | MTMS | 0,607 | 0,598 | 40 | 150 | 4 | 71,2 |
| 9 | 0,324 | DMTMS | 0,501 | 0,498 | 40 | 150 | 4 | 55,3 |
| 10 | 0,335 | NB | 0,798 | 0,335 | 40 | 150 | 4 | 47,5 |
| 11 | 0,345 | DMS | 0,60 | 0,765 | 40 | 150 | 4 | 35,0 |
| 12 | 0,32 | TMS | 0,578 | 0,58 | 40 | 140 | 3 | 36,5 |
| 13 | 0,164 | TMS | 0,59 | 0,595 | 60 | 150 | 3 | 47,7 |
| 14 | 0,32 | TMS | 0,578 | 0,578 | 40 | 160 | 2 | 69,5 |

## Exemples 15 à 17

On a préparé divers esters d'éthyle (R COOC$_2$H$_5$) par réaction de l'éthylène sur un acide carboxylique (R COOH), selon le processus général décrit précédemment, en opérant dans la tétraméthylènesulfone en présence d'acide trifluorométhanesulfonique, comme catalyseur. Les conditions particulières et les résultats obtenus après 4 heures de réaction sont donnés dans le tableau III ci-après :

TABLEAU III

| N° Exemple | $CF_3SO_3H$ Mole/litre | TMS Mole | Acide carboxylique Nature | Acide carboxylique Mole | T en °C | P en bars | TT % en $RCOOC_2H_5$ |
|---|---|---|---|---|---|---|---|
| 15 | 0,306 | 0,603 | propionique | 0,6 | 150 | 40 | 83,7 |
| 16 | 0,295 | 0,50 | hexanoïque | 0,497 | 160 | 20 | 72,4 |
| 17 | 0,308 | 0,613 | benzoïque | 0,4 | 140 | 40 | $\simeq$ 100,0 |

### Exemples 18 à 22

Selon le processus général décrit précédemment on a réalisé divers essais portant sur la préparation de l'acétate d'éthyle par réaction de l'éthylène (pression : 40 bars) sur l'acide acétique dans un milieu renfermant des proportions variables de tétraméthylène sulfone, le catalyseur étant l'acide trifluorométhanesulfonique. La température de réaction était de 150° C et la durée de réaction, de 4 heures.

Les conditions particulières ainsi que les résultats obtenus sont portés dans le tableau IV ci-après, dans lequel f désigne le rapport du nombre de moles de solvant au nombre total de moles de solvant et d'acide carboxylique.

TABLEAU IV

| N° Exemple | Catalyseur Mole/litre | Solvant Mole | Acide acétique Mole | f | TT % |
|---|---|---|---|---|---|
| 18 | 0,293 | 0,163 | 1,437 | 0,102 | 14,0 |
| 19 | 0,317 | 0,502 | 1,005 | 0,333 | 37,3 |
| 4 | 0,326 | 0,60 | 0,60 | 0,50 | 75,0 |
| 20 | 0,311 | 0,802 | 0,35 | 0,696 | $\simeq$ 100,0 |
| 21* | 0,09 | 0,899 | 0,105 | 0,895 | 50,2 |
| 22** | 0,319 | 0,60 | 0,60 | 0,5 | 52,0 |

\* Cet essai a été réalisé à 110 °C.

\*\* Cet essai a été réalisé avec une pression de 20 bars d'éthylène.

### Exemple 23

Dans une bombe de 250 cm³, en Hastelloy C, on charge 44,4 g d'acide acétique, 93,3 g de tétraméthylène sulfone et 7,0 g d'acide trifluorométhane sulfonique.

On porte à la température de 150° C au moyen d'un four à agitation longitudinale. On envoie alors de l'éthylène sous une pression de 40 bars. Au bout de 3 heures on arrête la réaction.

On distille alors l'acétate d'éthyle formé sous pression réduite. On obtient :

45,0 g d'acétate d'éthyle et 111,8 g de pied de distillation renfermant essentiellement de la tétraméthylène sulfone, de l'acide trifluorométhanesulfonique et l'acide acétique n'ayant pas réagi.

### Exemples 24 à 29

En utilisant le mode opératoire décrit pour l'exemple 23, mais en chargeant le pied de distillation (111,8 g) obtenu dans l'exemple 23, on réalise une seconde opération dans les mêmes conditions de température, de pression et de durée, en ajoutant 35,5 g d'acide acétique frais. En fin d'opération, on distille l'acétate d'éthyle formé et l'on utilise le pied de distillation pour réaliser l'opération de l'exemple 25 ; les opérations correspondant aux exemples 24 à 29 sont réalisées selon ce même principe.

Dans le tableau I ci après, on a porté pour chaque exemple la quantité de pied de distillation recyclée, en provenance de l'opération précédente (recyclage), la quantité d'acide acétique frais ajouté (acide), ainsi que la masse d'acétate d'éthyle distillé (M) en fin de chaque opération.

**0 003 205**

TABLEAU I

| Exemples | 24 | 25 | 26 | 27 | 28 | 29 |
|---|---|---|---|---|---|---|
| Acide (g) | 35,5 | 32,5 | 31,8 | 27,2 | 28,7 | 29,1 |
| Recyclage (g) | 111,8 | 114,1 | 115,6 | 119,7 | 116,9 | 115,7 |
| M (g) | 46,1 | 42,9 | 38,5 | 41,2 | 41,0 | 39,5 |

**Revendications**

1) Procédé de préparation d'esters d'éthyle en phase liquide à partir d'éthylène sous pression et d'au moins un acide carboxylique, en présence d'une quantité efficace d'un catalyseur qui est un acide minéral ou un acide organique fort, éventuellement sous la forme d'un ester d'alcoyle inférieur, caractérisé en ce que l'on opère en présence d'au moins un solvant dissociant, inerte et stable, et capable d'augmenter l'acidité du milieu.

2) Procédé selon 1, caractérisé en ce que le solvant a une constante diélectrique au moins de l'ordre de 25.

3) Procédé selon la revendication 1 ou 2 caractérisé en ce que l'acide carboxylique est choisi dans le groupe constitué par les acides aliphatiques ayant jusqu'à 20 atomes de carbone dans la molécule, saturés ou insaturés, pouvant porter un ou plusieurs atomes d'halogènes dans la molécule, les acides benzoïque, toluique, naphténique, succinique, adipique et phtalique.

4) Procédé selon la revendication 1 ou 2 caractérisé en ce que l'acide carboxylique est choisi parmi les acides acétique, propionique, butyrique, isobutyrique, hexanoïque, monochloracétique, dichloracétique, bromacétique et chloropropionique.

5) Procédé selon la revendication 1 ou 2 caractérisé en ce que l'acide carboxylique est l'acide acétique.

6) Procédé selon la revendication 1 ou 2 caractérisé en ce que le solvant est choisi parmi la tétraméthylène sulfone et ses dérivés, et leurs mélanges.

7) Procédé de préparation de l'acétate d'éthyle par réaction en phase liquide de l'éthylène sous pression, sur un mélange renfermant de l'acide acétique au moins un catalyseur acide, et au moins un solvant choisi dans le groupe constitué par la tétraméthylène sulfone et ses dérivés caractérisé en ce que, en fin d'opération on sépare l'acétate d'éthyle formé par tout moyen approprié, en vue de sa récupération, et on recycle le mélange renfermant essentiellement le catalyseur, le solvant et, éventuellement, l'acide acétique qui n'a pas réagi.

8) Procédé selon la revendication 1, 2 ou 7 caractérisé en ce que le catalyseur est choisi dans le groupe constitué par l'acide sulfurique, les acides alcanesulfoniques ayant au plus 6 atomes de carbone dans la molécule, l'acide benzènesulfonique, l'acide p-toluènesulfonique, les acides benzènedisulfoniques, les acides naphtalènedisulfoniques, les acides perfluoroalcanesulfoniques ayant au plus 8 atomes de carbone, éventuellement sous forme de leurs esters d'alcoyles inférieurs, pris isolément ou en mélange.

9) Procédé selon la revendication 1, 2 ou 7 caractérisé en ce que le catalyseur est choisi parmi les acides perfluoroalcanesulfoniques ayant au plus 8 atomes de carbone dans la molécule, leurs esters d'alcoyles inférieurs, pris isolément ou en mélange.

10) Procédé selon la revendication 1, 2 ou 7 caractérisé en ce que le catalyseur est choisi dans le groupe constitué par l'acide trifluorométhanesulfonique, ses esters d'alcoyles inférieurs, pris isolément ou en mélange.

11) Procédé selon la revendication 8, 9 ou 10 caractérisé en ce que le catalyseur est partiellement ou totalement sous la forme d'un ester dont le radical alcoyle contient de 1 à 4 atomes de carbone.

12) Procédé selon la revendication 8, 9 ou 10 caractérisé en ce que le catalyseur est partiellement ou totalement sous la forme d'un ester d'éthyle.

13) Procédé selon la revendication 1, 2, 6 ou 7 caractérisé en ce que la fraction molaire f du solvant est supérieure ou égale à 0.4.

14) Procédé selon la revendication 1, 2 ou 7 caractérisé en ce que la quantité de catalyseur est comprise entre 0,05 et 2 moles par litre de mélange.

15) Procédé selon la revendication 14, caractérisé en ce que la quantité de catalyseur est comprise entre 0,1 et 1 mole par litre de mélange.

16) Procédé selon la revendication 1, 2 ou 7 caractérisé en ce que la pression d'éthylène est comprise entre 20 et 60 bars.

17) Procédé selon la revendication 1, 2 ou 7 caractérisé en ce que la température de réaction est comprise entre 100 et 200° C.

18) Procédé selon la revendication 17 caractérisé en ce que la température de réaction est comprise entre 130 et 160° C.

6

# 0 003 205

**Claims**

1. Process for the preparation of ethyl esters, in the liquid phase, from ethylene under pressure and at least one carboxylic acid, in the presence of an effective amount of a catalyst, which is a mineral acid or a strong organic acid, optionally in the form of a lower alkyl ester, characterised in that the reaction is carried out in the presence of at least one inert and stable, dissociating solvent capable of increasing the acidity of the medium.

2. Process according to 1, characterised in that the solvent has a dielectric constant of the order of at least 25.

3. Process according to Claim 1 or 2, characterised in that the carboxylic acid is chosen from the group comprising saturated or unsaturated aliphatic acids having up to 20 carbon atoms in the molecule, which can carry one or more halogen atoms in the molecule, and benzoic, toluic, naphthenic, succinic, adipic and phthalic acids.

4. Process according to Claim 1 or 2, characterised in that the carboxylic acid is chosen from amongst acetic, propionic, butyric, isobutyric, hexanoic, monochloroacetic, dichloroacetic, bromoacetic and chloropropionic acids.

5. Process according to Claim 1 or 2, characterised in that the carboxylic acid is acetic acid.

6. Process according to Claim 1 or 2, characterised in that the solvent is chosen from amongst tetramethylenesulphone and its derivatives, and mixtures thereof.

7. Process for the preparation of ethyl acetate by reacting, in the liquid phase, ethylene under pressure and a mixture containing acetic acid, at least one acid catalyst and at least one solvent chosen from the group comprising tetramethylenesulphone and its derivatives, characterised in that, at the end of the operation, the ethyl acetate formed is separated off by any suitable means, in order to be recovered, and the mixture essentially containing the catalyst, the solvent and, if appropriate, the unreacted acetic acid is recycled.

8. Process according to Claim 1, 2 or 7, characterised in that the catalyst is chosen from the group comprising sulphuric acid, alkanesulphonic acids having at most 6 carbon atoms in the molecule, benzenesulphonic acid, p-toluenesulphonic acid, benzenedisulphonic acids, naphthalenedisulphonic acids and perfluoroalkanesulphonic acids having at most 8 carbon atoms , optionally in the form of their lower alkyl esters, taken in isolation or in a mixture.

9. Process according to Claim 1, 2 or 7, characterised in that the catalyst is chosen from amongst perfluoroalkanesulphonic acids having at most 8 carbon atoms in the molecule, and their lower alkyl esters, taken in isolation or in a mixture.

10. Process according to Claim 1, 2 or 7, characterised in that the catalyst is chosen from the group comprising trifluoromethanesulphonic acid and its lower alkyl esters, taken in isolation or in a mixture.

11. Process according to Claim 8, 9 or 10, characterised in that the catalyst is partially or totally in the form of an ester in which the alkyl radical contains from 1 to 4 carbon atoms.

12. Process according to Claim 8, 9 or 10, characterised in that the catalyst is partially or totally in the form of an ethyl ester.

13. Process according to Claim 1, 2, 6 or 7, characterised in that the molar fraction f of the solvent is greater or equal to 0.4.

14. Process according to Claim 1, 2 or 7, characterised in that the amount of catalyst is between 0.05 and 2 mols per litre of mixture.

15. Process according to Claim 14, characterised in that the amount of catalyst is between 0.1 and 1 mol per litre of mixture.

16. Process according to Claim 1, 2 or 7, characterised in that the ethylene pressure is between 20 and 60 bars.

17. Process according to Claim 1, 2 or 7, characterised in that the reaction temperature is between 100 and 200° C.

18. Process according to Claim 17, characterised in that the reaction temperature is between 130 and 160° C.

**Ansprüche**

1. Verfahren zur Herstellung von Äthylestern in flüssiger Phase ausgehend von Äthylen unter Druck und mindestens einer Carbonsäure, in Gegenwart einer wirksamen Menge eines Katalysators, der eine anorganische Säure oder eine starke organische Säure ist, gegebenenfalls in Form eines niederen Alkylesters, dadurch gekennzeichnet, daß man in Gegenwart mindestens eines dissozierenden Lösungsmittels arbeitet, das inert und beständig und in der Lage ist, die Azidität des Mediums zu erhöhen.

2. Verfahren nach Anspruch 1, dadurch gekennzeichnet, daß das Lösungsmittel eine Dielektrizitätskonstante von mindestens 25 aufweist.

3. Verfahren nach Anspruch 1 oder 2, dadurch gekennzeichnet, daß die Carbonsäure ausgewählt wird aus der Gruppe, bestehend aus den gesättigten oder undesättigten aliphatischen Carbonsäuren mit bis zu 20 Kohlenstoffatomen im Molekül, die ein oder mehrere Halogenatome im Molekül aufweisen

7

können, sowie Benzoesäure, Toluylsäure, Naphtensäure, Bernsteinsäure, Adipinsäure und Phthalsäure.

4. Verfahren nach Anspruch 1 oder 2, dadurch gekennzeichnet, daß die Carbonsäure ausgewählt wird aus der Gruppe der Essigsäure, Propionsäure, Buttersäure, Isobuttersäure, Hexansäure, Monochloressigsäure, Dichloressigsäure, Bromessigsäure und Chlorpropionsäure.

5. Verfahren nach Anspruch 1 oder 2, dadurch gekennzeichnet, daß die Carbonsäure Essigsäure ist.

6. Verfahren nach Anspruch 1 oder 2, dadurch gekennzeichnet, daß das Lösungsmittel unter Tetramethylensulfon und seinen Derivaten sowie Gemischen davon ausgewählt wird.

7. Verfahren zur Herstellung von Äthylacetat mittels Umsetzung in flüssiger Phase von Äthylen unter Druck mit einem Gemisch, das Essigsäure und mindestens einen sauren Katalysator enthält, und mindestens ein Lösungsmittel aus der Gruppe bestehend aus Tetramethylensulfon und seinen Derivaten, dadurch gekennzeichnet, daß man am Ende der Umsetzung das entstandene Äthylacetat auf beliebig geeignete Weise abtrennt und isoliert und das Gemisch, das im wesentlichen den Katalysator, das Lösungsmittel und gegebenenfalls nicht umgesetzte Essigsäure enthält, rückführt.

8. Verfahren nach Anspruch 1, 2 oder 7, dadurch gekennzeichnet, daß der Katalysator ausgewählt wird aus der Gruppe bestehend aus Schwefelsäure, den Alkansulfonsäuren mit höchstens 6 Kohlenstoffatomen im Molekül, Benzolsulfonsäure, p-Toluosulfonsäuren, Benzoldisulfonsäuren, Naphthalindisulfonsäuren, Perfluoralkansulfonsäuren mit höchstens 8 Kohlenstoffatomen, gegebenenfalls in Form ihrer niederen Alkylester, einzeln oder im Gemisch miteinander.

9. Verfahren nach Anspruch 1, 2 oder 7, dadurch gekennzeichnet, daß der Katalysator ausgewählt wird aus den Perfluoralkansulfonsäuren, die höchstens 8 Kohlenstoffatome im Molekül aufweisen und/oder ihren niedern Alkylestern.

10. Verfahren nach Anspruch 1, 2 oder 7, dadurch gekennzeichnet, daß der Katalysator ausgewählt wird aus der Gruppe Trifluormethansulfonsäure und/oder deren niederen Alkylestern.

11. Verfahren nach Anspruch 8, 9 oder 10, dadurch gekennzeichnet, daß der Katalysator teilweise oder vollständig in Form eines Esters vorliegt, dessen Alkylgruppe 1 bis 4 Kohlenstoffatome enthält.

12. Verfahren nach Anspruch 8, 9 oder 10, dadurch gekennzeichnet, daß der Katalysator teilweise oder vollständig in Form eines Äthylesters vorliegt.

13. Verfahren nach Anspruch 1, 2, 6 oder 7, dadurch gekennzeichnet, daß der Molanteil f des Lösungsmittels gleich ist 0,4 oder darüber.

14. Verfahren nach Anspruch 1, 2 oder 7, dadurch gekennzeichnet, daß die Menge des Katalysators 0,05 bis 2 Mol je Liter Gemisch beträgt.

15. Verfahren nach Anspruch 14, dadurch gekennzeichnet, daß die Menge des Katalysators 0,1 bis 1 Mol je Liter Gemisch beträgt.

16. Verfahren nach Anspruch 1, 2 oder 7, dadurch gekennzeichnet, daß der Äthylendruck 20 bis 60 bar beträgt.

17. Verfahren nach Anspruch 1, 2 oder 7, dadurch gekennzeichnet, daß die Reaktionstemperatur bei 100 bis 200° C liegt.

18. Verfahren nach Anspruch 17, dadurch gekennzeichnet, daß die Reaktionstemperatur 130 bis 160° C beträgt.